Europäisches Patentamt

European Patent Office

Office européen des brevets

(11) Publication number: **0 292 003 B1**

# EUROPEAN PATENT SPECIFICATION

(45) Date of publication of patent specification: **23.03.94**   (51) Int. Cl.5: **A61L 2/04**, A61L 2/18, A61L 2/08

(21) Application number: **88108131.9**

(22) Date of filing: **20.05.88**

(54) **Stabilzation of biological and pharmaceutical products during inactivation of viral and bacterial contaminants.**

(30) Priority: **22.05.87 US 52926**

(43) Date of publication of application:
**23.11.88 Bulletin 88/47**

(45) Publication of the grant of the patent:
**23.03.94 Bulletin 94/12**

(84) Designated Contracting States:
**AT BE CH DE ES FR GB GR IT LI LU NL SE**

(56) References cited:
| | |
|---|---|
| **EP-A- 0 035 204** | **EP-A- 0 037 078** |
| **EP-A- 0 053 338** | **EP-A- 0 077 870** |
| **EP-A- 0 117 064** | **EP-A- 0 124 044** |
| **EP-A- 0 177 836** | **EP-A- 0 196 761** |
| **EP-A- 0 199 479** | **US-A- 4 379 085** |
| **US-A- 4 470 968** | |

**THE LANCET, 06.07.1985**

**D.L. ARONSON: "Virus transmission by factor VIII products", pp. 301-323**

(73) Proprietor: **ARMOUR PHARMACEUTICAL PRODUCTS, INC.**
**Delaware Corporate Center I,**
**1 Righter Parkway, Suite 114,**
**Talleyville**
**Wilmington, Delaware 19803(US)**

(72) Inventor: **Feldman, Fred**
**407 Gettysburg Street**
**Park Forest Illinois(US)**
Inventor: **Klekamp, Mark S.**
**703 Heritage Drive**
**Bourbonnais Illinois(US)**
Inventor: **Hrinda, Michael E.**
**9718 Swift Creek Court**
**Fairfax Station Virginia(US)**
Inventor: **Shaw, Arthur B.**
**2718 Hemlock Avenue**
**Alexandria Virginia(US)**
Inventor: **Chandra, Sudhish**
**884 South McKinley Avenue**
**Kankakee Illinois(US)**

(74) Representative: **Brauns, Hans-Adolf, Dr. rer.**
**nat. et al**
**Hoffmann, Eitle & Partner**
**Patentanwälte**
**Postfach 81 04 20**
**D-81904 München (DE)**

**Description**

This invention relates to a process of stabilizing biological and pharmaceutical products in order to protect their activity during treatment designed to inactivate viral and/or bacterial contaminants. More particularly, the invention relates to the stabilization of proteinaceous materials that are to be rendered free of biological contaminants such as viruses and the like, and especially Epstein-Barr Virus (EBV) and cytomegalovirus (CMV), infectious hepatitis viruses, such as hepatitis B virus (HBV), non-A non-B hepatitis virus (NANBV), and the Human Immunodeficiency Virus (HTLV III/ LAV/HIV) which causes AIDS.

Biological and pharmaceutical products may be contaminated, or are suspect of being contaminated by viruses and bacteria which render them unsuitable and often quite dangerous for therapeutic use. Such contamination can be a source of contagious diseases posing great risk to the users of such products.

It is possible to inactivate the viral and bacterial contaminants in the initial or intermediate stages of production or in the final products. The inactivation procedures, however, are very harsh and usually result in a substantial loss of activity of labile biologicals and pharmaceuticals. Therefore, prior to inactivation of the contaminants, it is necessary to stabilize the biologicals and pharmaceuticals to protect their activity.

In its general aspect, this invention relates to the stabilization of biological and pharmaceutical products in the liquid or suspension state while rendering them free of viral and bacterial contaminants using thermal, chemical or irradiation methods of inactivation.

In a specific aspect, the invention relates to the production of biological and pharmaceutical products to be pasteurized in the liquid state during the manufacturing or purification process.

Liquid pasteurization is an established method for inactivating pathogenic microorganisms which contaminate products derived from biological materials, such as human plasma. Specifically, this invention relates to the use of stabilizers which prevent inactivation of proteins and allow pasteurization to be performed with minimal loss of biological and therapeutic activity. Stabilization is achieved by the addition of appropriate amounts of one or more mono-, di-, or tri-saccharides or sugar alchohols in combination with one or more neutral salts prior to pasteurization.

Agents derived from biological sources designed for therapeutic, prophylactic or diagnostic use constitute a large and important segment of products used in health care. Examples of such products and uses thereof include the following: Factor VIII is used to treat hemophelia; immune serum globulin is employed in the treatment of congenital gamma globulin deficiency, poliomyletitis and hepatitis A and B; antithrombin III is a coagulation inhibitor; plasminogen-streptokinase complex is used in the treatment of thromboembolism; and plasma growth hormone corrects pituitary growth deficiency.

An important concern associated with the use of therapeutic agents derived from biological sources is the transmission of disease, especially viral disease. Prevalent viral contaminants include hepatitis B virus (HBV), non-A, non-B hepatitis virus (NANBV), and HTLV III/LAV/HIV, which cause AIDS. In order to ensure that products produced from biological sources are virus-safe, various methodologies have been proposed for virus inactivation. These, in general, can be summarized as methods of: heating of the blood products in aqueous solution, if desired, with the addition of virucidal substances; heating of the blood products in aqueous solution in the presence of stabilizing agents; treating the blood products with organic solvents; irradiating the blood products in the solid state; and heating of the blood products in the dry state.

All these methods aim at destroying the potential viral and bacterial infectivity of the preparations while substantially maintaining their desired biological activity.

Representative methods of the prior art believed to be relevant to the present invention are as follows:

USP No. 4,456,590 (Rubenstein) relates to a method of heat treatment of plasma fractions in lyophilized form for the purpose of inactivating hepatitis virus that may be present in the fractions.

USP No. 4,314,997 (Shanbrom) teaches a chemical inactivation process by which plasma and plasma derivatives in solution or suspension are contacted with a non-denaturing amphiphile, i.e., a surface active agent, to cause irreversible destruction of endotoxins and inactivation of hepatitis viruses.

USP No. 4,440,679 (Fernandes et al.) describes a method wherein therapeutically active proteins are pasteurized by mixing the protein composition with a pasteurization-stabilizing amount of a polyol prior to pasteurization.

USP No. 4,297,344 (Schwinn et al.) discloses a process for the stabilization against heat of the coagulation Factors II, VIII, XIII, antithrombin III and plasminogen, in aqueous solution, which comprises adding to the solution both an aminoacid and one or more of a monosaccharide, an oligosaccharide or a sugar alcohol.

USP No. 4,585,654 (Landaburu et al.) pertains to a process of inactivating viruses in plasma protein solutions by heating the same in the presence of a polyol, a surface active agent and a chelating agent.

3

Mitra et al. (US-A-4,470,968) describe a process for pasteurizing coagulation Factors II, VII, IX and X in the presence of a stabilizing amount of a polyol and a source of citrate ions.

EP-A-0 077 870 describes the virus inactivation of Factor VIII solutions by heating in the presence of a principal stabilizer of neutral amino acids, monosaccharides, polysaccharides, or sugar alcohols and an auxiliary stabilizer of carboxylic acid salts having 3-10 carbon atoms.

EP-A-0 177 836 describes virus inactivation of immunoglobulins by heating in a dry state in the presence of glycine, sodium chloride, sodium acetate (alkali metal chlorides and acetates), PEG, albumin, or mannitol.

EP-A-0 196 761 describes a process for inactivating viruses in solutions of gamma-globulin by heating an aqueous solution in the presence of a monosaccharide, disaccharide, or sugar alcohol, and an auxiliary stabilizer selected from neutral amino acids, neutral inorganic acid salts, salts of organic carboxylic acids (exemplifying only those having 3-15 carbon atoms), and surface agents. The only specific neutral salts taught by the reference are the alkaline halides, sodium chloride, potassium chloride, and magnesium chloride.

EP-A-0 053 338 describes virus inactivation of preparations of Factors IX and/or X by warming the preparation in the presence of calcium ions and an amino acid and/or a saccharide or sugar alcohol.

EP-A-0 037 078 describes a process for heat treatment of Factor XIII in the presence of a principal stabilizer consisting of neutral amino acids, monosaccharides, and sugar alcohols and an auxiliary stabilizer consisting of salts of carboxylic acids having 3-10 carbon atoms.

It appears, according to some investigators, that wet heating of certain biological products is more effective than dry heating. For example, in a comparison of wet heating against dry heat treatment for safety from NANBV transmission, results suggested that wet heating provided a higher degree of safety (Kernoff et al., The Lancet, Sept. 28, 1985, p. 721). Clinical data shows that Factor VIII concentrate pasteurized in a liquid phase transmitted no HBV, NANBV, or HTLV III/LAV/HIV in more than six years of clinical experience. (International Congress of the World Federation of Hemophelia, June 8-13 (1986); N. Heimburger: "Preparation of a F VIII concentrate free from pathogenic viruses.") The efficacy of liquid pasteurization in virus inactivation is perhaps best demonstrated by the history of serum albumin, where no case of serum hepatitis has been traced to transfusion of heated albumin over a period of more than thirty five years. ("Milestones in Blood Transfusion and Immunohaemotology", Vox Sang 46:338-340 (1984)).

Direct demonstration of viral inactivation by pasteurization in the liquid state has shown that cytomegalovirus, Epstein-Barr virus, herpes simplex virus, poliovirus, vaccinia virus, hepatitis B virus, non-A non-B hepatitis virus, and HTLV III/LAV/HIV are all inactivated, and further, that neoantigens are not formed during liquid-state pasteurization ("Pasteurization as an Efficient Method to Inactivate Blood Borne Viruses in Factor VIII Concentrates", J. Hilfenhous and E. Weidman; Arzneim.-Forsch/Drug Res. 36(I) Nr. 4 (1986)).

Given that liquid pasteurization is a proven means of inactivating viral contaminants, it becomes necessary to stabilize thermally sensitive bioactive molecules from heat inactivation. Stabilization against the thermal inactivation of these products is achieved through the use of one or more mono-, di-, or tri-saccharides, or sugar alcohols in combination with one or more neutral salts.

This method significantly differs from the closest resembling methods of the prior art references described above. To wit:

(a) It differs from the method of using the combination of monosaccharides, oligosaccharides or sugar alcohols with amino acids or certain amino acid analogs by the replacement of amino acids and their analogs with a neutral salt. There are distinct advantages associated with this replacement. First, the addition of stabilizing amounts of amino acids to a biological or pharmaceutical product may cause inactivation due to abrupt and often significant pH changes. This effect is minimized or eliminated through the use of stabilizing salts. Second, many amino acids are sparingly soluble in aqueous solvents whereas most stabilizing salts are quite soluble, allowing the addition of higher concentration stabilizers. Third, when using amino acids as stabilizers it is necessary to remove all residual stabilizer from the final product as intensive therapy with a pharmaceutical product containing residual amino acid could overload the body's detoxification system in patients with severe liver or kidney damage.

(b) It also differs from the method which claims to use a polyol as the sole stabilizer. Pasteurization using only a polyol in the absence of any glycine results in relatively poor recovery of, for example, Factor VIII activity. It is also apparent that the final formulations of the reference products contain glycine and, therefore, the pasteurization conditions resemble those using a sugar in combination with an amino acid.

The present invention relates to a method of inactivating pathogens in a biological or a pharmaceutical material comprising the steps of mixing the material in an aqeuous solution containing at least one primary stabilizer selected from sugars or sugar alcohols and at least one secondary stabilizer selected from the group consisting of neutral salts, adjusting the pH of the aqueous solution to about 5.0 to 10.0, subjecting the aqueous solution to a pathogen inactivating process, and optionally removing the primary and secondary stabilizers from the aqueous solution, characterized in that the secondary stabilizer is selected from the group consisting of sodium acetate, potassium acetate, lithium acetate, magnesium acetate, ammonium acetate, barium acetate, sodium sulfate, ammonium sulfate, lithium sulfate, barium sulfate, potassium sulfate and magnesium sulfate.

More specifically, the invention provides a method of inactivating pathogens in a biological or pharmaceutical material by mixing the material in aqueous solution with one or more primary stabilizer selected from the groups consisting of mono-, di-, or tri-saccharides or sugar alcohols having a concentration of 10% w/v to saturation, adding one or more secondary stabilizers selected from the group consisting of the above salts at concentrations of 0.01M to saturation to obtain a mixture, thermally inactivating pathogens by maintaining a temperature of 30°C to 100°C for 1 minute to 72 hours, adjusting the pH to a range of 6.7 to 7.7, and removing said primary and secondary stabilizers after thermal inactivation.

In accordance with the present invention, biological and pharmaceutical products are pasteurized in the presence of primary and secondary stabilizers, the combined effect of which insures that the products' activity is preserved during the heating process.

The heat treatment is carried out at a temperature and for a period of time sufficient to inactivate the pathogens, especially infectious viruses, but at the same time to retain the activity of the products. Such heat treatment is maintained for about 1 minute to 72 hours at a temperature of 30°C to 100°C, preferably for about 10 to 20 hours at 55°C to 70°C, and most preferably for 10 hours at 60°C.

In freeing the biological/pharmaceutical products from pathogens, an aqueous medium is employed in which are dissolved or suspended the primary and secondary stabilizers. The product to be pasteurized is contacted with the medium and subjected to pasteurizing conditions sufficient to deactivate pathogens. Treatment of the product can be carried out at any stage in the production process, such as the starting material stage or at some later step in the production sequence, or with certain products, after completion of the production process.

Following the addition of the stabilizers, the pH is adjusted, if necessary, to a range of 5.0 to 10.0 and preferably to 6.7 to 7.7. Product concentration in the aqueous medium will generally range from about 1 mg/ml to about 500 mg/ml and more preferably from about 1 to 100 mg/ml.

After pasteurization is complete, the stabilizers are removed by appropriate means which include: dialysis (McPhie, P. (1971), Methods in Enzymol. 22, pp. 23-32); diafiltration (Blatt, W. F. et al. (1968) Anal. Biochem. 26, p. 151); column chromatography (Porath, J. and Flodin, P. (1959), Nature 183, pp. 1657-1659); precipitation (Dixon, M. and Webb, E. C. (1961), Adv. in Protein Chem. 16, pp. 197-219, Academic Press, N.Y.; Honig, W. and Kula, M. R. (1976) Anal. Biochem. 72, pp. 502-512); or any other method by which removal of the stabilizers can be effected.

Products

The process of the present invention is applicable to a great number and variety of materials and products in the biomedical and pharmaceutical fields intended to be used in the human or animal body for biomedical or therapeutic purposes as well as non-therapeutic experimental purposes. Contemplated materials and products which can be made free of pathogens using the process of the present invention include but are not limited to:

blood fractions such as antihemophilic factor (Smith, J. K. and Bidwell, E. (1979) Clinics in Hoematol. 8, pp.184-205); prothrombin complex, i.e., Factors II, VII, IX, and X (Chandra, S. and Brummelhuis, H.G.J. (1981) Vox Sang. 41, pp. 257-273); Protein C (Stenflo, J. (1976) J. Biol. Chem. 251, pp. 355-363 and Bajaj, S. P. et al. (1983) Prep. Biochem. 13, pp. 191-214); Protein S (DiScipio, R. G., et al. (1977) Biochemistry 16, pp. 698-706); Antithrombin III (Rosenberg, R.D., and Damus, P.S. (1973) J. Biol Chem. 248, pp. 6490-6505); C-1 esterase inhibitor (Heimburger, N. (1974) Bayer Symposium V "Proteinase Inhibitors", pp. 14-22, Springer-Verlag); alpha 1 antitrypsin (Heimburger, N. supra.); Fibronectin (Mosesson, M. N. and Amrani, D. L. (1980), Blood 56 pp. 145-158); Gamma Globulin (Oncley et al. (1949) J. Amer. Chem. Soc. 71, pp. 541-550); biological or pharmaceutical products derived from human or animal origin such as insulin, enzymes, coenzymes, antibodies and hormones; biological or pharmaceutical products derived from human or animal placentae such as blood fractions and vaccines; biological or pharmaceutical products derived by recombinant DNA techniques and produced in bacteria, fungi, or mammalian cell culture systems (Vane, J. and Cuatrecases, P. (1984), Nature 312, pp. 303-305 and Maniatis, T. et al. (1982), Molecular Cloning: A Laboratory Manual, Cold Spring Harbor, N.Y.). These products and materials are available from various commercial sources or can be produced by using well-known preparative techniques. For example, blood fractions and blood proteins can be obtained from human blood plasma by fractionation according to known techniques such as, for example, the alcohol fractionation of Cohn described in U.S. Pat. No. 2,390,074 and the Journal of the American Chemical Society Vol. 68, p. 459 (1946). These methods as well as other techniques are summarized in "The Plasma Proteins", second edition, Vol. III, pp. 548-550, Academic Press, New York, N.Y. (1977).

Primary Stabilizers

The primary stabilizers include mono-, di-, or tri-saccharides, and sugar alcohols, e.g., glucose, sucrose, xylose, fructose and mannitol, glucosaminitol, sorbitol, galactosaminitol and the like. One or more of these stabilizers are used in the process of the present invention at a concentration range of 10% w/v to saturation.

The preferred primary stabilizer is sucrose at a concentration of about 50% to 70% w/v.

Secondary Stabilizers

The secondary stabilizers consist of certain neutral salts and are used in the process of the present invention at a concentration of 0.01M to saturation. The stabilizing salts are defined for the purposes of this invention as neutral salts of common organic and mineral acids selected from sodium acetate, ammonium acetate, potassium acetate, sodium phosphate, sodium sulfate, and ammonium sulfate, lithium sulfate, potassium sulfate, magnesium sulfate, barium sulfate, lithium acetate, magnesium acetate, and barium acetate.

The concentration of the stabilizing salt to be used is dependent upon two parameters. First, it depends upon the solubility of the molecule, and second, the concentration at which salting out of the protein(s) begins to occur in the solution.

The following examples will further illustrate the invention although it will be understood that the invention is not limited thereto.

EXAMPLE 1

Pasteurization of a Low-Fibrinogen AHF Concentrate Stabilization by Sucrose + Sodium Acetate

Each of three vials of Factorate-LF® (low-fibrinogen Factor VIII produced by Armour Pharmaceutical Company, Kankakee, IL) was reconstituted in 10 ml of water for injection. To one was added 1.0 g sucrose per ml of solution plus 0.5 g sodium acetate per ml of solution. To another was added 1.0 g sucrose per ml of solution. No stabilizers were added to the third vial. The pH of the three solutions was adjusted to 7.0 using a small amount of 0.5N hydrochloric acid. The solutions were heated at 60°C for 10 hours. Samples before and after pasteurization from each solution were assayed for procoagulant activity (F.VIII:C) by the one stage Activated Partial Thromboplastin Time (APTT) method which is essentially the same as the methods described by Hardisty, R. M. and MacPherson, J. C. (1962), Thrombosis et Diathesis Haemorrhagica 7, pp. 215-229 and Zacharski, L. R. and Rosenstein, R. (1978), Amer. J. Clin. Path. 70, pp. 280-286. The results are summarized in Table 1.

## Table 1

### F.VIII Units/ml

| Stabilizers | Before Pasteurization | After Pasteurization | % Recovery |
|---|---|---|---|
| Sucrose + Sodium Acetate: | 11.4 | 9.2 | 80.7 |
| Sucrose: | 14.2 | 5.9 | 41.5 |
| None: | 20.1 | 0 | 0 |

EXAMPLE 2

Pasteurization of Highly Purified Factor VIII:C Stabilization by Sucrose + Sodium Acetate

Monoclate® (Factor VIII:C, produced by Armour Pharmaceutical Company, Kankakee, IL) was pasteurized in the following manner. Three aliquots of 10 ml each (approximately 500 units) of ultrafiltered eluate from an anti-F.-VIII:R-Sepharose-C1-2B column were used. The first was mixed with 1.0 g sucrose per ml of solution plus 0.5 g sodium acetate per ml of solution. To the second aliquot was added 1.0 g sucrose per ml of solution. No stabilizers were added to the third aliquot. The pH of the three solutions was adjusted to 7.0 with a small amount of 0.5N hydrochloric acid. The solutions were heated at 60°C for 10 hours. Samples before and after pasteurization were assayed for procoagulant activity (F.VIII:C) by the one stage APTT method referred to in Example 1. The results are summarized in Table 2.

## Table 2

### F.VIII Units/ml

| Stabilizers | Before Pasteurization | After Pasteurization | % Recovery |
|---|---|---|---|
| Sucrose + Sodium Acetate: | 17.6 | 10.4 | 60.2 |
| Sucrose: | 23.2 | 6.7 | 28.9 |
| None: | 23.0 | 1.0 | 4.0 |

EXAMPLE 3

Pasteurization of Highly Purified Factor VIII:C Stabilization by Sucrose + Sodium Sulfate

Monoclate® (Factor VIII:C, produced by Armour Pharmaceutical Company, Kankakee, IL) was pasteurized in the following manner. Two aliquots of 10 ml each (approximately 500 units) of ultrafiltered eluate from an anti-F.VIII:R-Sepharose-C1-2B column were used. The first was mixed with 1.0 g sucrose per ml of solution plus 0.28 g sodium sulfate per ml of solution. To the second aliquot was added 1.0 g sucrose per ml of solution. The pH of the two solutions was adjusted to 7.0 with a small amount of 0.5N hydrochloric acid. The solutions were heated at 60°C for 10 hours. Samples before and after pasteurization were assayed for procoagulant activity (F.VIII:C) by the one stage APTT method referred to in Example 1. The results are summarized in Table 3.

## Table 3

### F.VIII Units/ml

| Stabilizers | Before Pasteurization | After Pasteurization | % Recovery |
|---|---|---|---|
| Sucrose + Sodium Sulfate: | 21.5 | 17.6 | 81.9 |
| Sucrose: | 20.0 | 5.7 | 28.5 |

EXAMPLE 4

Pasteurization of a Low-Fibrinogen AHF Concentrate Stabilization by Sucrose + Various Neutral Salts

Each of nine vials of Factorate-LF® (low-fibrinogen Factor VIII produced by Armour Pharmaceutical Company, Kankakee, IL) was reconstituted in 10 ml of water for injection. After reconstitution was completed, 1.0 g sucrose per ml of solution was added to each. Different stabilizing salts were then added, at concentrations of 1-2M, to each of the Factorate-LF®/sucrose solutions. The salts used were: potassium acetate (2.0g), ammonium acetate (1.6g), sodium sulfate (2.8g), ammonium sulfate (2.6g), sodium chloride (1.2g), ammonium chloride (1.1g), magnesium chloride (0.95g), choline chloride (2.8g), and sodium phosphate, dibasic (1.4 g). The pH of the solutions was then adjusted to 7.0 using either 0.5N hydrochloric acid or 0.5N sodium hydroxide, except for the solution containing sodium phosphate. The pH of this solution was 8.2, unadjusted. The solutions were heated at 60°C for 10 hours. Samples before and after pasteurization from each solution were assayed for procoagulant activity (F.VIII:C) by the one stage APTT method referred to in Example 1. The results are summarized in Table 4.

## Table 4

| Stabilizers | % Recovery of F.VIII After Pasteurization |
|---|---|
| Sucrose + Potassium Acetate | 89.4 |
| Sucrose + Ammonium Acetate | 72.7 |
| Sucrose + Sodium Sulfate | 89.1 |
| Sucrose + Ammonium Sulfate | 78.6 |
| Sucrose + Sodium Chloride | 16.7 |
| Sucrose + Ammonium Chloride | 34.7 |
| Sucrose + Magnesium Chloride | 10.9 |
| Sucrose + Choline Chloride | 10.0 |
| Sucrose + Sodium Phosphate (Dibasic) | 28.0 |

The following conclusions are readily apparent from the tests described by the examples and the results shown in the Tables:

pasteurization of the proteinaceous materials at 60°C for 10 hours results in close to complete destruction of their activity; while the use of a sugar alone (sucrose) improves the retention of activity, it is considerably less effective than when a combination of a sugar and certain of the stabilizing salts are employed as stabilizers and; the combination of a sugar with certain stabilizing salts as stabilizers is superior to the combination of a sugar and other stabilizing salts.

Other Embodiments

In accordance with another embodiment of the present invention, stabilization of biological or pharmaceutical products can also be obtained when methods other than pasteurization are used for bacterial and viral inactivation. These methods include: chemical inactivation, such as, using ozone as the pathogen deactivating agent, as described in USP No. 4,632,980, using an organic compound to promote deactivation as described in USP No. 4,640,834; or using high energy (UV, infrared, and gamma) irradiation as described by USP No. 2,897,123.

It is apparent that numerous modifications and variations of the invention may be made without departing from the spirit and scope thereof. The specific embodiments described are given by way of example only, and the invention is limited only by the scope of the appended claims.

## Claims

1. A method of inactivating pathogens in a biological or a pharmaceutical material comprising the steps of: mixing the material in an aqueous solution containing at least one primary stabilizer selected from sugars or sugar alcohols and at least one secondary stabilizer selected from neutral salts, adjusting the pH of the aqueous solution to about 5.0 to 10.0, subjecting the aqueous solution to a pathogen inactivating process, and optionally removing the primary and secondary stabilizers from the aqueous solution, characterized in that the secondary stabilizer is selected from the group consisting of sodium acetate, potassium acetate, lithium acetate, magnesium acetate, ammonium acetate, barium acetate, sodium sulfate, ammonium sulfate, lithium sulfate, barium sulfate, potassium sulfate and magnesium sulfate.

2. The method of Claim 1 wherein the pathogen inactivating process comprises subjecting said aqueous solution to a temperature of 30°C to 100°C for about 1 minute to 72 hours.

3. The method of Claim 1 wherein the pathogen inactivating process comprises chemical inactivation or irradiation.

4. The method of Claim 1 wherein the pH of the aqueous solution is adjusted to about 6.7 to about 7.7 and the solution is subjected to pasteurization conditions sufficient to deactivate pathogens therein.

5. The method of Claim 4 wherein the pasteurization is carried out at a temperature of about 55°C to 70°C for 10 to 20 hours.

6. The method of any of Claims 1 to 5 wherein the primary stabilizer is a mono-, di-, tri-saccharide.

7. The method of Claim 6 wherein the sugars are glucose, sucrose, xylose, fructose, mannitol, and said sugar alcohols are galactitol, glucosaminitol, sorbitol or galactosaminitol.

8. The method of any of Claims 1 to 7 wherein the primary stabilizer is present at a concentration from 10% w/v to saturation.

9. The method of Claim 8 wherein the stabilizer is sucrose at a concentration of about 50% w/v to 70% w/v.

10. The method of any of Claims 1 to 9, wherein the secondary stabilizer is present in a concentration of about .01M to saturation.

11. The method of any of Claims 1 to 10 wherein the secondary stabilizer is present in a concentration of about 0.5M to about 2.5M.

12. The method of any of Claims 1 to 11 wherein removal of primary and secondary stabilizers is by diafiltration, dialysis, column chromatography or by precipitation.

13. The method of any of Claims 1 to 12 wherein said material is in a processing stage of the finished product, an active compound in the finished product, or the finished product.

14. The method of any of Claims 1 to 13 wherein said material is Factor VIII or prothrombin complex.

15. A proteinaceous product having been treated at any desired stage of its production by a method which comprises the method of any of Claims 1 to 14.

## Patentansprüche

1. Verfahren zur Inaktivierung von Pathogenen in einem biologischen oder pharmazeutischen Material, umfassend die Schritte: Vermischen des Materials in wäßriger Lösung, die mindestens einen primären Stabilisator, ausgewählt aus Zuckern oder Zuckeralkoholen, und mindestens einem sekundären Stabilisator, ausgewählt aus Neutralsalzen, enthält, Einstellen des pH-Werts der wäßrigen Lösung auf ca. 5,0 bis 10,0, Einwirken eines Pathogen-inaktivierenden Prozesses auf die wäßrige Lösung, und wahlweise Entfernen der primären und sekundären Stabilisatoren aus der wäßrigen Lösung, dadurch gekennzeichnet, daß der sekundäre Stabilisator ausgewählt ist aus Natriumacetat, Kaliumacetat, Lithiumacetat, Magnesiumacetat, Ammoniumacetat, Bariumacetat, Natriumsulfat, Ammoniumsulfat, Lithiumsulfat, Bariumsulfat, Kaliumsulfat und Magnesiumsulfat.

2. Verfahren nach Anspruch 1, worin der Pathogen-inaktivierende Prozeß eine Hitzebehandlung der wäßrigen Lösung bei einer Temperatur von 30°C bis 100°C für ca. 1 min bis 72 h umfaßt.

3. Verfahren nach Anspruch 1, worin der Pathogen-inaktivierende Prozeß chemische Inaktivierung oder Bestrahlung umfaßt.

4. Verfahren nach Anspruch 1, worin der pH-Wert der wäßrigen Lösung auf ca. 6,7 bis ca. 7,7 eingestellt wird, und die Lösung Pasteurisierungsbedingungen unterworfen wird, die ausreichen, die darin enthaltenen Pathogene zu deaktivieren.

5. Verfahren nach Anspruch 4, worin die Pasteurisierung bei einer Temperatur von ca. 55°C bis 70°C für 10 bis 20 h durchgeführt wird.

6. Verfahren nach einem der Ansprüche 1 bis 5, worin der primäre Stabilisator ein Mono-, Di-, Trisaccharid ist.

7. Verfahren nach Anspruch 6, worin die Zucker sind: Glukose, Saccharose, Xylose, Fructose, Mannitol; und die Zuckeralkohole sind: Galactitol, Glucosaminitol, Sorbitol oder Galactosaminitol.

8. Verfahren nach einem der Ansprüche 1 bis 7, worin der primäre Stabilisator in einer Konzentration von 10 Gew./Vol.-% bis zur Sättigung vorliegt.

9. Verfahren nach Anspruch 8, worin der Stabilisator Saccharose in einer Konzentration von ca. 50 Gew./Vol.-% bis 70 Gew./Vol.-% ist.

10. Verfahren nach einem der Ansprüche 1 bis 9, worin der zweite Stabilisator in einer Konzentration von ca. 0,01 M bis zur Sättigung vorliegt.

11. Verfahren nach einem der Ansprüche 1 bis 10, worin der sekundäre Stabilisator in einer Konzentration von ca. 0,5 M bis ca. 2,5 M vorliegt.

12. Verfahren nach einem der Ansprüche 1 bis 11, worin das Entfernen der primären und sekundären Stabilisatoren durch Diafiltration, Dialyse, Säulenchromatographie oder durch Präzipitation erfolgt.

13. Verfahren nach einem der Ansprüche 1 bis 12, worin das Material sich in einem Herstellungstadium des Endprodukts befindet, ein Wirkstoff im Endprodukt oder das Endprodukt selbst ist.

14. Verfahren nach einem der Ansprüche 1 bis 13, worin das Material Faktor VIII oder Prothrombinkomplex ist.

15. Proteinartiges Produkt, welches in einem erwünschten Herstellungsstadium nach einem Verfahren entsprechend einem der Ansprüche 1 bis 14 behandelt wurde.

**Revendications**

1. Procédé pour inactiver des agents pathogènes dans un matériau biologique ou pharmaceutique, qui comprend les étapes consistant à mélanger le matériau dans une solution aqueuse contenant au moins un stabilisant primaire choisi parmi les sucres ou les alcools de sucre et au moins un stabilisant secondaire choisi parmi les sels neutres, à ajuster le pH de la solution aqueuse à une valeur d'environ 5,0 à 10,0, à soumettre la solution aqueuse à un processus d'inactivation des agents pathogènes et, éventuellement à éliminer, de la solution aqueuse, les stablisants primaires et secondaires, caractérisé en ce que le stabilisant secondaire est choisi dans le groupe comprenant l'acétate de sodium, l'acétate de potassium, l'acétate de lithium, l'acétate de magnésium, l'acétate d'ammonium, l'acétate de baryum, le sulfate de sodium, le sulfate d'ammonium, le sulfate de lithium, le sulfate de baryum, le sulfate de potassium et le sulfate de magnésium.

2. Procédé selon la revendication 1, dans lequel le processus d'inactivation des agents pathogènes consiste à soumettre la solution aqueuse à une température comprise entre 30 °C et 100 °C pendant environ 1 minute à 72 heures.

3. Procédé selon la revendication 1, dans lequel le processus d'inactivation des agents pathogènes comprend une inactivation ou une irradiation chimique.

4. Procédé selon la revendication 1, dans lequel le pH de la solution aqueuse est ajusté à une valeur d'environ 6,7 à environ 7,7 et la solution est soumise à des conditions de pasteurisation suffisantes pour y désactiver les agents pathogènes.

5. Procédé selon la revendication 4, dans lequel la pasteurisation est mise en oeuvre à une température d'environ 55 °C à 70 °C pendant 10 à 20 heures.

6. Procédé selon l'une quelconque des revendications 1 à 5, dans lequel le stabilisant primaire est un mono-, un di- ou un tri-saccharide.

7. Procédé selon la revendication 6, dans lequel les sucres sont le glucose, le saccharose, le xylose, le fructose, le mannitol et les alcools de sucre sont le galactitol, le glucosaminitol, le sorbitol ou le galactosaminitol.

8. Procédé selon l'une quelconque des revendications 1 à 7, dans lequel le stabilisant primaire est présent à une concentration de 10 % m/v par rapport à la concentration à la saturation.

9. Procédé selon la revendication 8, dans lequel le stabilisant est le saccharose à une concentration d'environ 50 à 70 % m/v.

10. Procédé selon l'une quelconque des revendications 1 à 9, dans lequel le stabilisant secondaire est présent à une concentration d'environ 0,01M par rapport à la saturation.

11. Procédé selon l'une quelconque des revendications 1 à 10, dans lequel le stabilisant secondaire est présent à une concentration d'environ 0,5 à environ 2,5M.

12. Procédé selon l'une quelconque des revendications 1 à 11, dans lequel l'élimination des stabilisants primaires et secondaires s'effectue par diafiltration, dialyse, chromatographie sur colonne ou précipitation.

13. Procédé selon l'une quelconque des revendications 1 à 12, dans lequel ce matériau est, au cours d'un stade de traitement du produit fini, un principe actif du produit fini ou le produit fini proprement dit.

14. Procédé selon l'une quelconque des revendications 1 à 13, dans lequel le matériau est le facteur VIII ou le complexe prothrombique.

15. Produit protéiné qui a été traité, au cours de l'un des stades quelconques de sa production, par une méthode comprenant le procédé selon l'une quelconque des revendications 1 à 14.